# EUROPEAN PATENT APPLICATION

(11) **EP 3 992 274 A1**
(43) Date of publication of application: **04.05.2022**
(21) Application number: 20833479.7
(22) Date of filing: 23.06.2020
(51) Int. Cl.: C12M 1/00

(54) **CELL CLUSTER DIVIDER, CELL CLUSTER DIVIDER MANUFACTURING METHOD, AND CELL CLUSTER DIVIDING METHOD**

(30) Priority: 28.06.2019 US 201962868529 P
(71) Applicant: I Peace, Inc., Palo Alto, CA 94303 (US); FANUC CORPORATION, Oshino-mura Minamitsuru-gun Yamanashi 401-0597 (JP)
(72) Inventor: TANABE, Koji, Palo Alto, California 94303 (US); HIRAIDE, Ryoji, Kyoto-shi, Kyoto 615-8245 (JP); BAN, Kazunori, Minamitsuru-gun, Yamanashi 401-0597 (JP); KINOSHITA, Satoshi, Minamitsuru-gun, Yamanashi 401-0597 (JP)
(74) Representative: MacLean, Martin Robert
(86) International application number: PCT/JP2020/024542
(87) International publication number: WO 2020/262351

(57) **Abstract**

There is provided a cell mass dissociator including a serpentine flow path which is a flow path through which a cell mass flows.

## Description

### Technical Field

The present invention relates to a cell technique, a cell mass dissociator, a method for manufacturing a cell mass dissociator, and a method for dissociating a cell mass.

### Background Art

Embryonic stem cells (ES cells) are stem cells derived from early human or mouse embryos. ES cells have pluripotency that allow them to differentiate into any type of cells in a living body. Currently, human ES cells can be used for cell transplantation therapy for neumerous diseases such as Parkinson's disease, juvenile diabetes, and leukemia. However, there are also obstacles to ES cell transplantation. In particular, ES cell transplantation can elicit immunorejection similar to rejection response that follows unsuccessful organ transplantation. In addition, there are many criticisms and dissenting opinions from a moral point of view regarding use of ES cells derived by destroying human embryos.

Under such circumstances, Professor Shinya Yamanaka at Kyoto University succeeded in deriving induced pluripotent stem cells (iPS cells) by introducing four genes: OCT3/4, KLF4, c-MYC, and SOX2 into somatic cells. For this, Professor Yamanaka was awarded the 2012 Nobel Prize in Physiology or Medicine (for example, refer to Patent Documents 1 and 2) . iPS cells are ideal pluripotent cells without rejection responses or moral issues. Therefore, iPS cells are expected to be used for cell transplantation therapy.

### Citation List

### Patent Document

Patent Document 1: Japanese Patent No. 4183742
Patent Document 2: Patent Publication JP-A-2014-114997

### Summary

### Technical Problem

When iPS cells are cultured, a cell mass may be dissociated. In addition, without limitation to iPS cells, and when cells are cultured, a cell mass may be dissociated. Here, one objective of the present invention is to provide a cell mass dissociator that can efficiently dissociate a cell mass, a method for manufacturing a cell mass dissociator, and a method for dissociating a cell mass.

### Solution to Problem

According to an aspect of the present invention, there is provided a cell mass dissociator which includes a serpentine flow path which is a flow path through which a cell mass flows.

In the cell mass dissociator, an underflow may occur in a fluid that flows through the flow path.

In the cell mass dissociator, the flow path may be composed of a groove that is provided in a substrate and a lid that covers the groove.

In the cell mass dissociator, the flow path may be embedded with an embedding member.

In the cell mass dissociator, the flow path may be a hole that is provided in a solid member.

In the cell mass dissociator, the flow path may be closed from the outside air.

The cell mass dissociator may further include a fluid machine for causing the cell mass to flow through the flow path.

In addition, according to an aspect of the present invention, there is provided a cell mass dissociator including a flow path through which a cell mass flows, wherein the flow path has a narrow part and a wide part that is wider than the narrow part, and the flow path is able to be closed from the outside air.

In the cell mass dissociator, an underflow may occur in a fluid that flows through the flow path.

The cell mass dissociator may further include a member that is disposed in a wide part of the flow path and partially prevents a flow of a fluid in the flow path.

In the cell mass dissociator, the wide part may be approximately circular.

In the cell mass dissociator, the wide part may be approximately polygonal.

In the cell mass dissociator, the wide part may be approximately hexagonal.

In the cell mass dissociator, the flow path may be composed of a groove that is provided in a substrate and a lid that covers the groove.

In the cell mass dissociator, the flow path may be embedded with an embedding member.

In the cell mass dissociator, the flow path may be a hole that is provided in a solid member.

The cell mass dissociator may further include a fluid machine for causing the cell mass to flow through the flow path.

In addition, according to an aspect of the present invention, there is provided a method for manufacturing a cell mass dissociator including forming a serpentine flow path through which a cell mass flows.

In the method for manufacturing a cell mass dissociator, a groove may be provided in a substrate to form the flow path.

In the method for manufacturing a cell mass dissociator, a groove may be covered with a lid to form the flow path.

The method for manufacturing a cell mass dissociator may further include embedding the flow path with an embedding member.

In the method for manufacturing a cell mass dissociator, a hole may be provided in a solid member to form the flow path.

In the method for manufacturing a cell mass dissociator, the flow path may be formed by a lithography method.

In the method for manufacturing a cell mass dissociator, the flow path may be formed by an ablation method.

In the method for manufacturing a cell mass dissociator, the flow path may be formed by a 3D printer.

In the method for manufacturing a cell mass dissociator, the flow path may be formed by an optical shaping method.

In addition, according to an aspect of the present invention, there is provided a method for manufacturing a cell mass dissociator including forming a flow path having a narrow part and a wide part that is wider than the narrow part, which is a flow path through which a cell mass flows; and closing the flow path from the outside air.

The method for manufacturing a cell mass dissociator may further include providing a member that partially prevents a flow of a fluid in the flow path in the wide part of the flow path.

In the method for manufacturing a cell mass dissociator, the wide part may be approximately circular.

In the method for manufacturing a cell mass dissociator, the wide part may be approximately polygonal.

In the method for manufacturing a cell mass dissociator, the wide part may be approximately hexagonal.

In the method for manufacturing a cell mass dissociator, the flow path may be composed of a groove that is provided in a substrate and a lid that covers the groove.

In the method for manufacturing a cell mass dissociator, a groove may be provided in a substrate to form the flow path.

In the method for manufacturing a cell mass dissociator, a groove may be covered with a lid to form the flow path.

The method for manufacturing a cell mass dissociator may further include embedding the flow path with an embedding member.

In the method for manufacturing a cell mass dissociator, a hole may be provided in a solid member to form the flow path.

In the method for manufacturing a cell mass dissociator, the flow path may be formed by a lithography method.

In the method for manufacturing a cell mass dissociator, the flow path may be formed by an ablation method.

In the method for manufacturing a cell mass dissociator, the flow path may be formed by a 3D printer.

In the method for manufacturing a cell mass dissociator, the flow path may be formed by an optical shaping method.

In addition, according to an aspect of the present invention, there is provided a method for dissociating a cell mass including causing a cell mass to flow through a serpentine flow path and dissociating the cell mass.

In the method for dissociating a cell mass, an underflow may occur in a fluid containing the cell mass in the flow path.

In the method for dissociating a cell mass, the cell mass may be circulated through the flow path.

In the method for dissociating a cell mass, the cell mass may be reciprocated through the flow path.

In the method for dissociating a cell mass, the cell mass that flows through the flow path may be contained in a cryopreservation solution.

In the method for dissociating a cell mass, the dissociated cell mass may be cryopreserved.

The method for dissociating a cell mass may further include bringing the cell mass into contact with a cell detachment solution before the cell mass flows through the serpentine flow path.

In addition, according to an aspect of the present invention, there is provided a method for dissociating a cell mass including causing a cell mass to flow through a flow path of a cell mass dissociator which has the flow path through which a cell mass flows and in which the flow path has a narrow part and a wide part that is wider than the narrow part, and the flow path is able to be closed from the outside air, and dissociating the cell mass.

In the method for dissociating a cell mass, an underflow may occur in a fluid containing the cell mass in the flow path.

In the method for dissociating a cell mass, a member that partially prevents a flow of a fluid in the flow path may be provided in the wide part of the flow path.

In the method for dissociating a cell mass, the cell mass may be circulated through the flow path.

In the method for dissociating a cell mass, the cell mass may be reciprocated through the flow path.

In the method for dissociating a cell mass, the cell mass that flows through the flow path may be contained in a cryopreservation solution.

In the method for dissociating a cell mass, the dissociated cell mass may be cryopreserved.

The method for dissociating a cell mass may further include bringing the cell mass into contact with a cell detachment solution before the cell mass flows through the flow path.

### Advantageous Effects of Invention

According to the present invention, it is possible to provide a cell mass dissociator that can efficiently dissociate a cell mass, a method for manufacturing a cell mass dissociator, and a method for dissociating a cell mass.

### Brief Description of Drawings

Fig. 1 shows schematic views showing a cell mass dissociator according to a first embodiment.
Fig. 2 shows diagrams showing simulation results according to a first example of the first embodiment.
Fig. 3 is an image showing a cell mass dissociator according to a second example of the first embodiment.
Fig. 4 shows images of a cell mass before and after it passes through the cell mass dissociator according to the second example of the first embodiment.
Fig. 5 shows trace diagrams showing an underflow in a flow path of the cell mass dissociator according to the second example of the first embodiment.
Fig. 6 is an image showing a cell mass dissociator according to a third example of the first embodiment.
Fig. 7 shows trace diagrams showing an underflow in a flow path of the cell mass dissociator according to the third example of the first embodiment.
Fig. 8 is an image showing a cell mass dissociator according to a fourth example of the first embodiment.
Fig. 9 shows trace diagrams showing an underflow in a flow path of the cell mass dissociator according to the fourth example of the first embodiment.
Fig. 10 shows schematic views showing a cell mass dissociator according to a second embodiment.
Fig. 11 shows schematic views showing the cell mass dissociator according to the second embodiment.
Fig. 12 shows schematic views showing the cell mass dissociator according to the second embodiment.
Fig. 13 shows schematic views showing the cell mass dissociator according to the second embodiment.
Fig. 14 shows diagrams showing simulation results according to a first example of the second embodiment.
Fig. 15 is an image showing a cell mass dissociator according to a second example of the second embodiment.
Fig. 16 shows trace diagrams showing an underflow in a flow path of the cell mass dissociator according to the second example of the second embodiment.
Fig. 17 is an image showing a cell mass dissociator according to a third example of the second embodiment.
Fig. 18 shows trace diagrams showing an underflow in a flow path of the cell mass dissociator according to the third example of the second embodiment.
Fig. 19 is an image showing a cell mass dissociator according to a fourth example of the second embodiment.
Fig. 20 shows trace diagrams showing an underflow in a flow path of the cell mass dissociator according to the fourth example of the second embodiment.
Fig. 21 is an image showing a cell mass dissociator according to a fifth example of the second embodiment.
Fig. 22 shows trace diagrams showing an underflow in a flow path of the cell mass dissociator according to the fifth example of the second embodiment.
Fig. 23 is an image showing a cell mass dissociator according to a sixth example of the second embodiment.
Fig. 24 shows trace diagrams showing an underflow in a flow path of the cell mass dissociator according to the sixth example of the second embodiment.
Fig. 25 is an image showing a cell mass dissociator according to a seventh example of the second embodiment.
Fig. 26 shows trace diagrams showing an underflow in a flow path of the cell mass dissociator according to the seventh example of the second embodiment.
Fig. 27 is an image showing a cell mass dissociator according to an eighth example of the second embodiment.
Fig. 28 shows trace diagrams showing an underflow in a flow path of the cell mass dissociator according to the eighth example of the second embodiment.
Fig. 29 is an image showing a cell mass dissociator according to a ninth example of the second embodiment.
Fig. 30 shows trace diagrams showing an underflow in a flow path of the cell mass dissociator according to the ninth example of the second embodiment.

### Description of Embodiments

Hereinafter, embodiments of the present invention will be described. In the following description of the drawings, the same or similar parts are denoted with the same or similar reference numerals. However, the drawings are schematic. Therefore, specific sizes and the like should be determined in light of the following description. In addition, it goes without saying that the drawings include parts having different relationships and ratios with each other.

### (First embodiment)

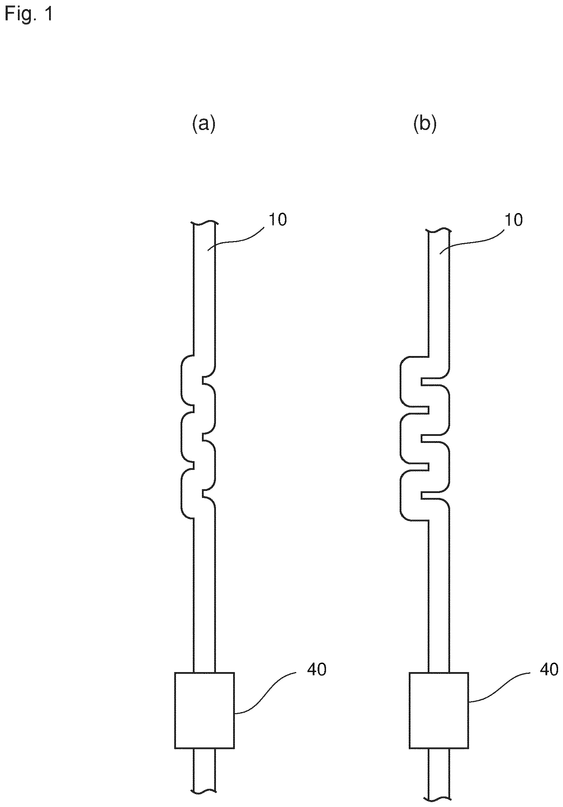

As shown in Fig. 1, a cell mass dissociator according to a first embodiment includes a flow path 10 through which a cell mass flows and which is a serpentine flow path 10. In the cell mass dissociator according to the first embodiment, the width of the serpentine flow path 10 is constant.

The flow path 10 may have a structure in which the inside can be closed from the outside air. The closed space including the inside of the flow path 10 may be configured to prevent exchange of a gas with the outside.

The flow path 10 may be formed with a groove provided in the substrate. The flow path 10 may be provided by providing a groove in a flat substrate and covering the groove with a lid. Alternatively, the flow path 10 may be formed by adhering substrates with grooves provided therein so that the positions of the grooves match. The groove forming the flow path 10 may be formed by, for example, an etching method, a lithography method, or a laser ablation method.

Alternatively, the flow path 10 may be formed of a tube. The flow path 10 may be enclosed and embedded in a non-gas-permeable substance.

In addition, alternatively, the flow path 10 may be a hole provided in the solid member. The hole may be provided in the solid member by a 3D printer. Examples of 3D printer methods include a material extrusion deposition method, a material jetting method, a binder jetting method and an optical shaping method.

The cell mass dissociator according to the first embodiment may further include a fluid machine 40 such as a pump for causing a cell mass to flow through the flow path. A positive displacement pump can be used as the fluid machine. Examples of positive displacement pumps include reciprocating pumps including a piston pump, a plunger pump, and a diaphragm pump, or rotary pumps including a gear pump, a vane pump, and a screw pump. Examples of diaphragm pumps include a turbing pump and a piezoelectric (piezo) pump. The turbing pump may be called a peristaltic pump. In addition, a microfluidic chip module in which various types of pumps are combined may be used. In the case where a sealable type pump such as a peristaltic pump, a turbing pump, or a diaphragm pump is used, it is possible to send the fluid without the pump coming into direct contact with the fluid inside the flow path.

The cell mass may be circulated through the flow path 10 or the cell mass may be reciprocated through the flow path 10. The cell mass may be brought into contact with a cell detachment solution before the cell mass flows through the flow path 10. Examples of cell detachment solutions include trypsin, TrypLE Select, Accutase, and EDTA. The cell mass that flows through the flow path 10 may be contained in the cryopreservation solution. In this case, the cell mass that is dissociated while flowing through the flow path 10 may be cryopreserved. The flow path 10 may be connected to a cryopreservation container.

According to the cell mass dissociator of the first embodiment, since the serpentine flow path 10 with a certain width causes an underflow to occur in a fluid that flows through the flow path 10, it is possible to efficiently dissociate the cell mass in the fluid such as a medium that flows through the flow path 10. Here, the underflow is one of, for example, a flow that causes a vortex, a turbulent flow, a reverse flow, a flow that generates parts with different flow speeds, a flow that generates a shear force, a flow that generates a friction force, a flow that rubs against objects, and a flow that creates parts in which flows traveling in different directions collide. In addition, according to the cell mass dissociator of the embodiment, for example, since the flow path 10 can be completely closed, it is possible to reduce a risk of cross-contamination due to leakage of cells from the flow path 10. In addition, for example, even if cells are infected with viruses such as HIV hepatitis viruses, it is possible to reduce an operator's risk of infection due to leakage of cells from the flow path 10. In addition, it is possible to reduce a risk of the fluid in the cell mass dissociator contaminating the air outside the cell mass dissociator with bacteria, viruses and fungi.

The type of cells forming the cell mass dissociated by the cell mass dissociator according to the first embodiment is not particularly limited. Cells forming the cell mass may be stem cells or differentiated cells. Examples of stem cells include ES cells and pluripotent stem cells (iPS cells). Examples of differentiated cells include nerve cells, retinal epithelial cells, hepatocytes, kidney cells, mesenchymal stem cells, blood cells, megakaryocytes, chondrocytes, cardiomyocytes, vascular cells, and epithelial cells. In addition, the cell mass may be a cell mass generated in a differentiation induction process, an embryoid body, an organoid, a spheroid, a cell mass of cell lines, or a cell mass of cancer cells.

### (First example of first embodiment)

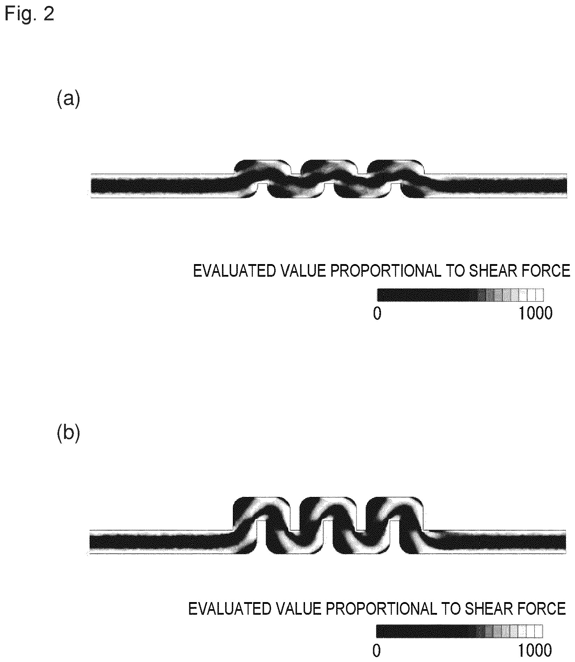

As shown in Fig. 2, shear force generated by a fluid that flowed through a serpentine flow path with a certain width was calculated by physics simulation. In Fig. 2, as the part had a lighter color, a stronger shear force was generated. Based on the results shown in Fig. 2, it was shown that a large shear force was generated near the side wall of the flow path. In addition, it was shown that, as compared with the linear part of the flow path, in the serpentine part, a large shear force was generated in a wide range.

### (Second example of first embodiment)

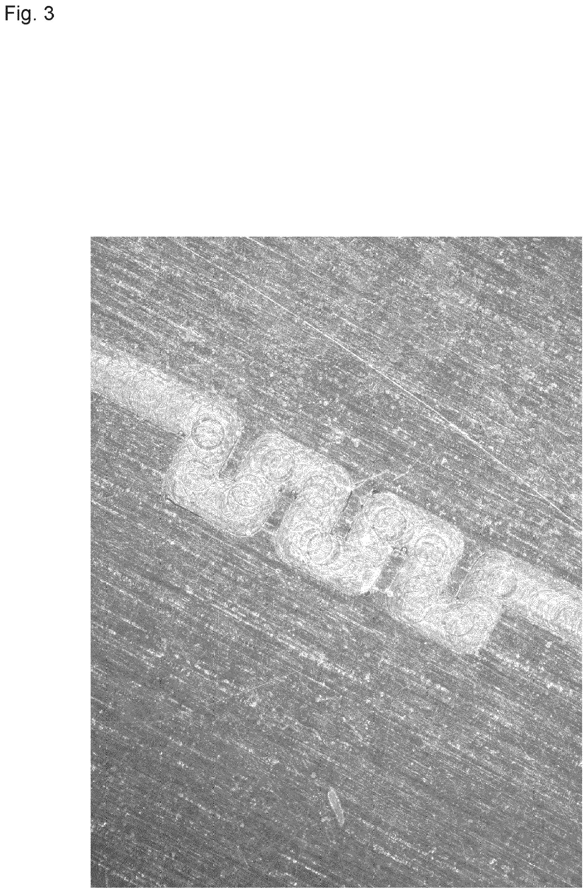

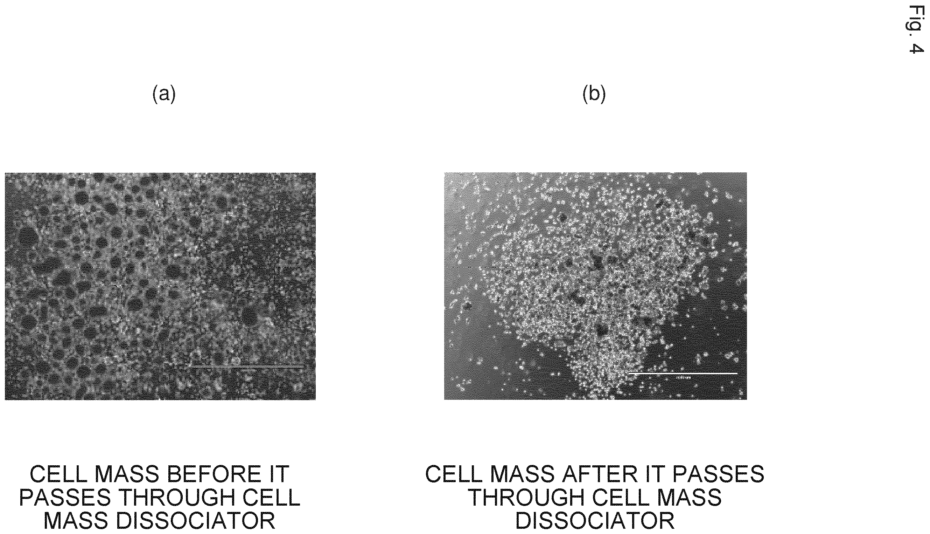

As shown in Fig. 3, a cell mass dissociator including a serpentine flow path with a certain width is produced. A groove forming the flow path was formed in a substrate by a laser ablation method. A glass plate was adhered to the substrate in which the groove was provided to form the flow path. Then, a medium containing a cell mass composed of iPS cells flowed through the flow path. As a result, as shown in Fig. 4, the cell mass composed of iPS cells was efficiently dissociated.

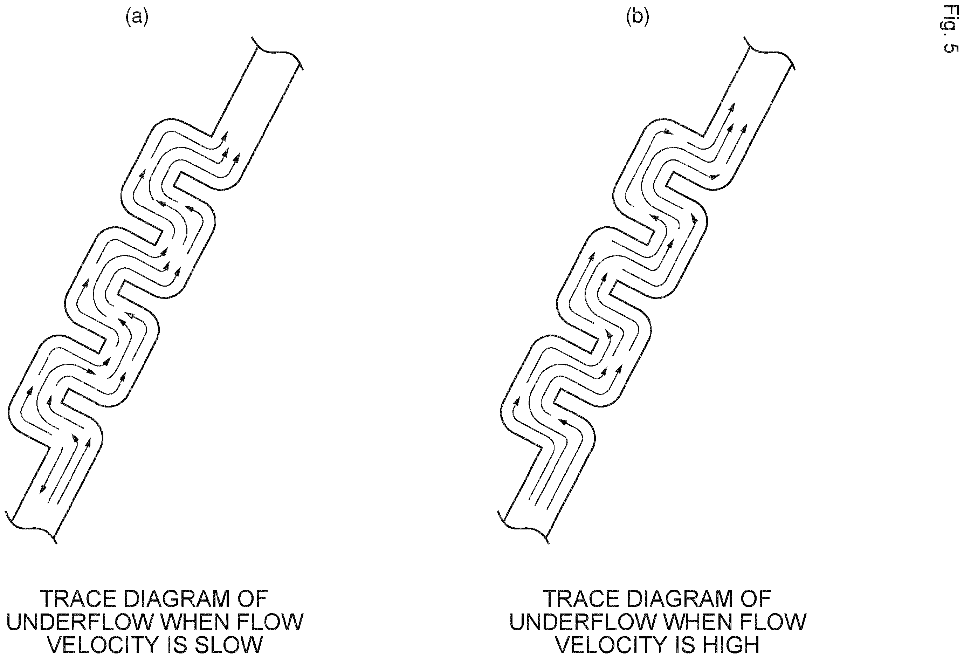

In addition, a liquid containing beads was poured into the cell dissociator shown in Fig. 3, and the movement of the beads was imaged. Fig. 5 shows a drawing in which the imaged movement of the beads was traced. It was shown that an underflow occurred in the serpentine flow path with a certain width according to the movement of the beads.

### (Third example of first embodiment)

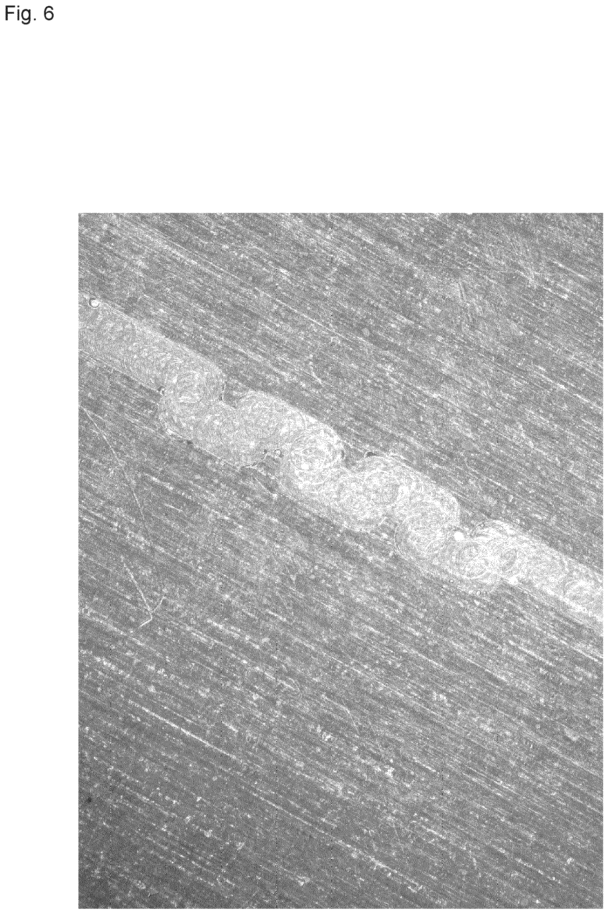

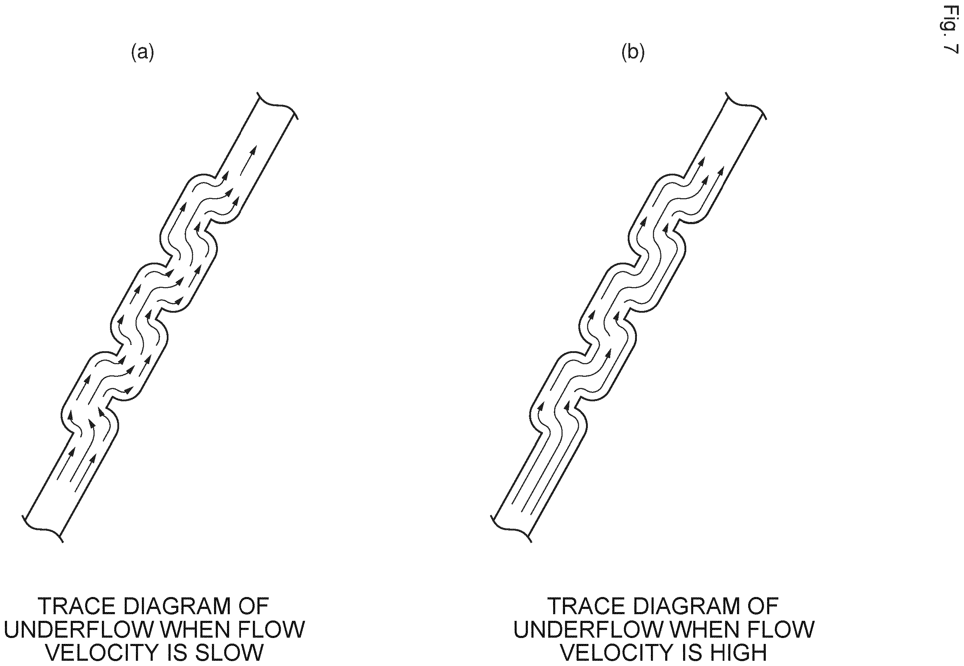

As shown in Fig. 6, a cell mass dissociator including a serpentine flow path with a certain width is produced. A liquid containing beads was poured into the cell dissociator shown in Fig. 6, and the movement of the beads was imaged. Fig. 7 shows a drawing in which the imaged movement of the beads was traced. It was shown that an underflow occurred in the serpentine flow path with a certain width according to the movement of the beads.

### (Fourth example of first embodiment)

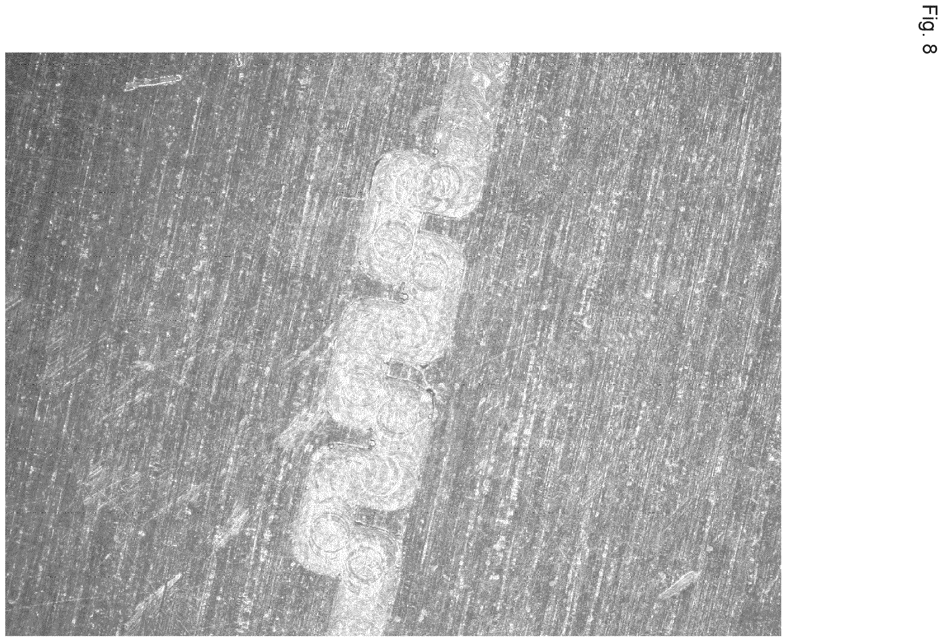

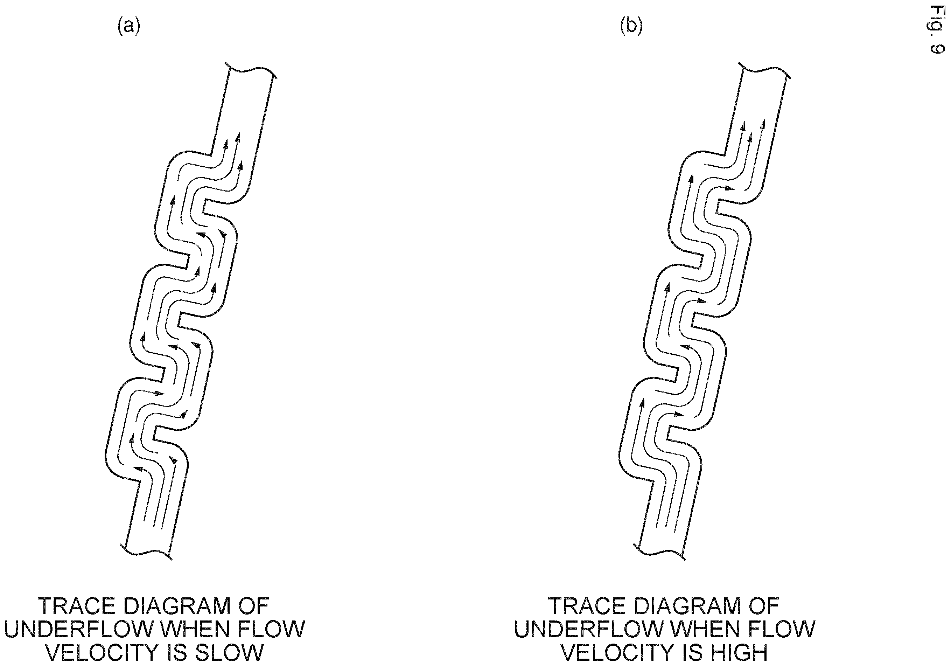

As shown in Fig. 8, a cell mass dissociator including a serpentine flow path with a certain width was produced. A liquid containing beads was poured into the cell dissociator shown in Fig. 8, and the movement of the beads was imaged. Fig. 9 shows a drawing in which the imaged movement of the beads was traced. It is seen that an underflow occurred in the serpentine flow path with a certain width according to the movement of the beads.

### (Second embodiment)

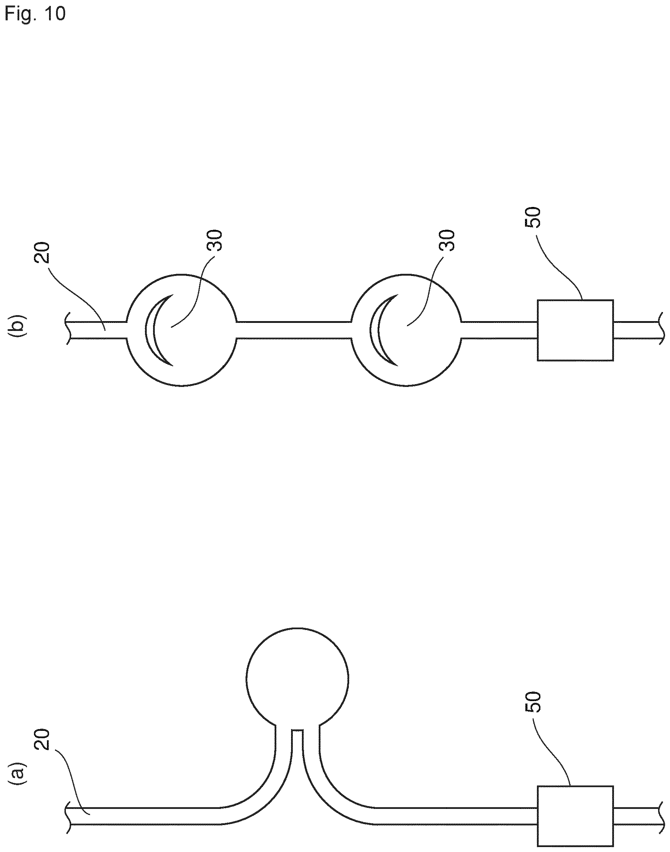

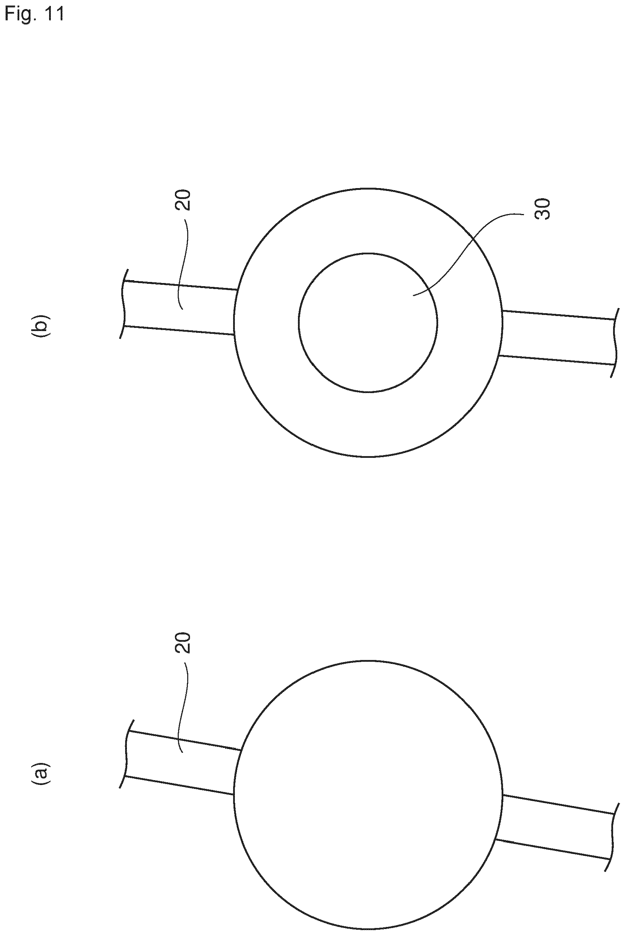

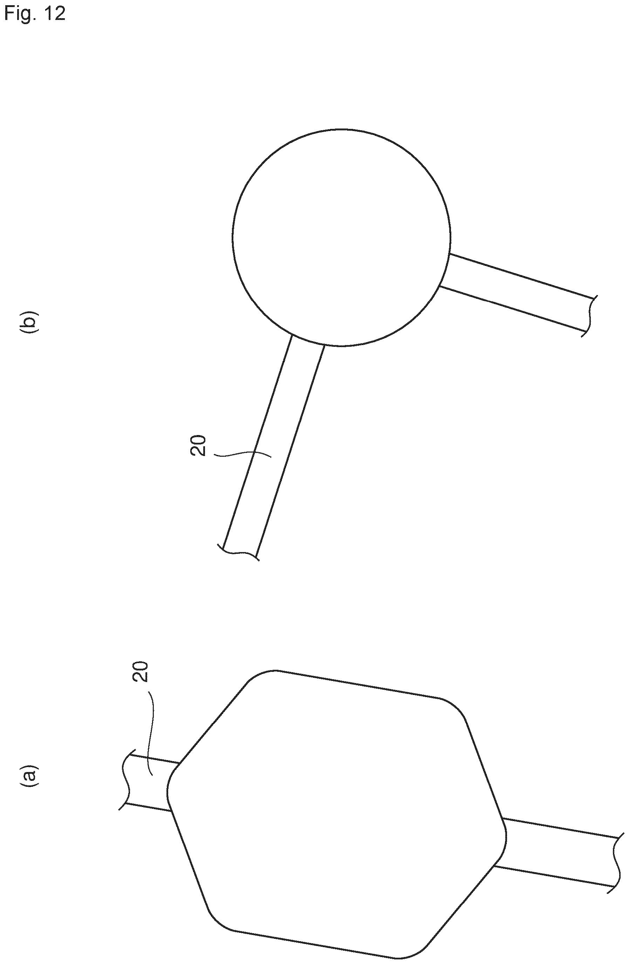

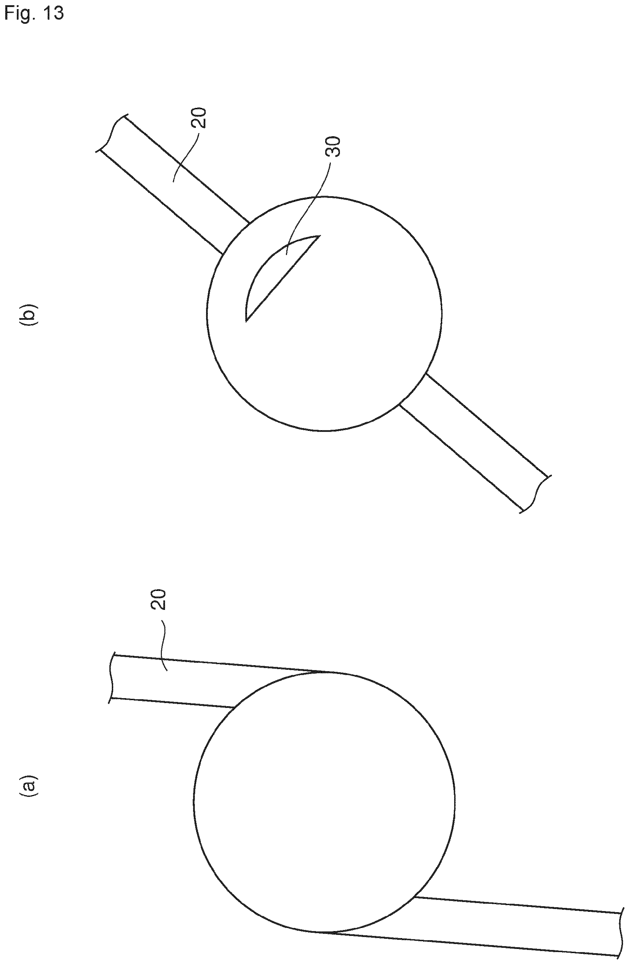

As shown in Fig. 10 to Fig. 13, a cell mass dissociator according to a second embodiment includes a flow path 20 through which a cell mass flows, the flow path 20 has a narrow part and a wide part that is wider than the narrow part, and the flow path 20 can be closed from the outside air. The closed space including the inside of the flow path 20 may be configured to prevent exchange of a gas with the outside.

The wide part may be approximately circular or approximately polygonal. The approximately polygon may be approximately hexagonal. As shown in Fig. 10(b), Fig. 11(b), and Fig. 13(b), the cell mass dissociator according to the second embodiment may further include a member 30 that is disposed in the wide part of the flow path 20 and partially prevents the flow of the fluid through the flow path 20. For example, the member 30 is disposed to face the direction of travel of the fluid that flows through the flow path 20. For example, at least a part of the member 30 is substantially perpendicular to the direction of travel of the fluid that flows through the flow path 20.

The flow path 20 may be formed with a groove provided in the substrate. The flow path 20 may be provided by providing a groove in a flat substrate and covering the groove with a lid. Alternatively, the flow path 20 may be formed by adhering substrates with grooves provided therein so that the positions of the grooves match. The groove forming the flow path 20 may be formed by, for example, an etching method, a lithography method, or a laser ablation method.

Alternatively, the flow path 20 may be formed of a tube. The flow path 20 may be enclosed and embedded in a non-gas-permeable substance.

In addition, alternatively, the flow path 20 may be a hole provided in the solid member. The hole may be provided in the solid member by a 3D printer. Examples of 3D printer methods include a material extrusion deposition method, a material jetting method, a binder jetting method and an optical shaping method.

The cell mass dissociator according to the second embodiment may further include a fluid machine 50 such as a pump for causing a cell mass to flow through the flow path as in the first embodiment.

The cell mass may be circulated through the flow path 20 or the cell mass may be reciprocated through the flow path 20. The cell mass may be brought into contact with a cell detachment solution before the cell mass flows through the flow path 20. The cell mass that flows through the flow path 20 may be contained in the cryopreservation solution. In this case, the cell mass that is dissociated while flowing through the flow path 20 may be cryopreserved. The flow path 20 may be connected to a cryopreservation container.

According to the cell mass dissociator of the second embodiment, since the flow path 20 having a narrow part and a wide part causes an underflow to occur in the fluid that flows through the flow path 20 and generates a shear force, it is possible to efficiently dissociate the cell mass in the fluid such as a medium that flows through the flow path 20. In addition, according to the cell mass dissociator of the embodiment, for example, since the flow path 20 can be completely closed, it is possible to reduce a risk of cross-contamination due to leakage of cells from the flow path 20. In addition, for example, even if cells are infected with viruses such as HIV hepatitis viruses, it is possible to reduce an operator's risk of infection due to leakage of cells from the flow path 20. In addition, it is possible to reduce a risk of the fluid in the cell mass dissociator contaminating the air outside the cell mass dissociator with bacteria, viruses and fungi.

Cells to be dissociated by the cell mass dissociator according to the second embodiment are not particularly limited as in the first embodiment. Examples of cells dissociated by the cell mass dissociator according to the second embodiment are the same those in the first embodiment.

### (First example of second embodiment)

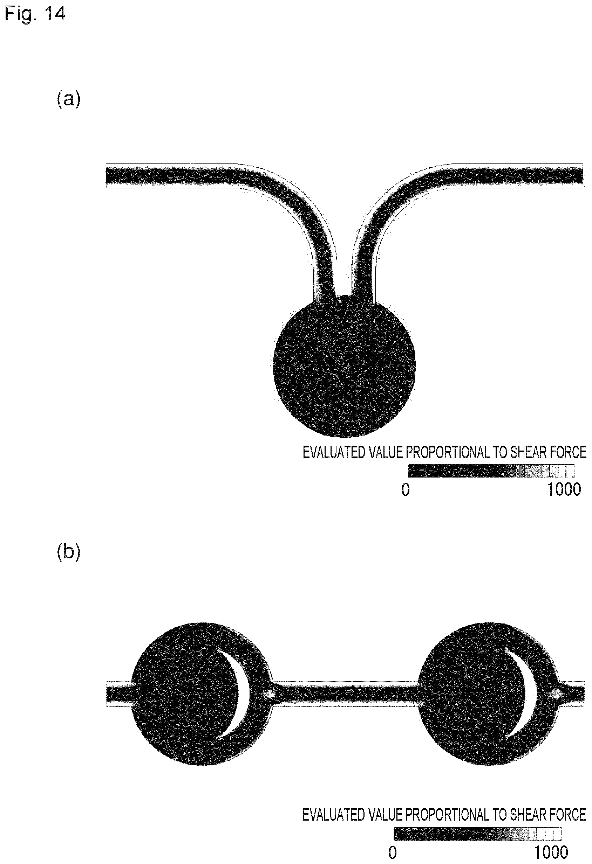

As shown in Fig. 14, a shear force generated by a fluid that flowed through a flow path having a narrow part and a wide part was calculated by physics simulation. In Fig. 14, as the part had a lighter color, a stronger shear force was generated. Based on the results shown in Fig. 14, it was shown that a large shear force was generated near the side wall of the flow path. In addition, it was shown that a large shear force was generated near the member that partially prevented the flow of the fluid in the flow path. In addition, it was shown that the fluid merged on the rear surface of the member with respect to the direction of travel of the fluid in the flow path, and a shear force was generated.

### (Second example of second embodiment)

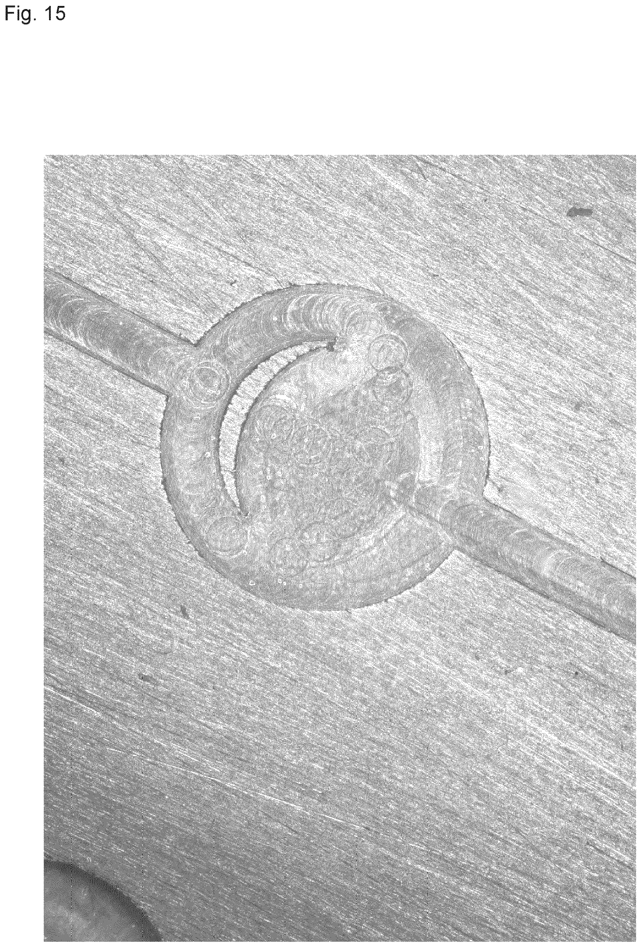

As shown in Fig. 15, a cell mass dissociator including a flow path which had a narrow part and a wide part and in which a member that partially prevented the flow of the fluid in the flow path was provided in the wide part was produced. A groove forming the flow path was formed in a substrate by a laser ablation method. A glass plate was adhered to the substrate in which the groove was provided to form the flow path. Then, a medium containing a cell mass composed of iPS cells flowed through the flow path. As a result, the cell mass composed of iPS cells was efficiently dissociated.

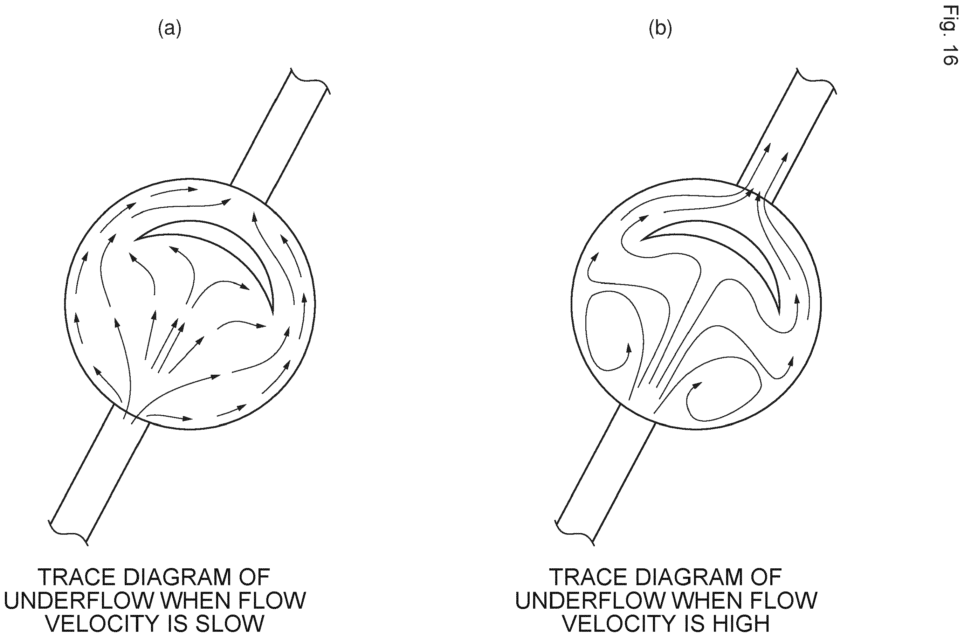

In addition, a liquid containing beads was poured into the cell dissociator shown in Fig. 15, and the movement of the beads was imaged. Fig. 16 shows a drawing in which the imaged movement of the beads was traced. It was shown that an underflow occurred in the serpentine flow path with a certain width according to the movement of the beads.

### (Third example of second embodiment)

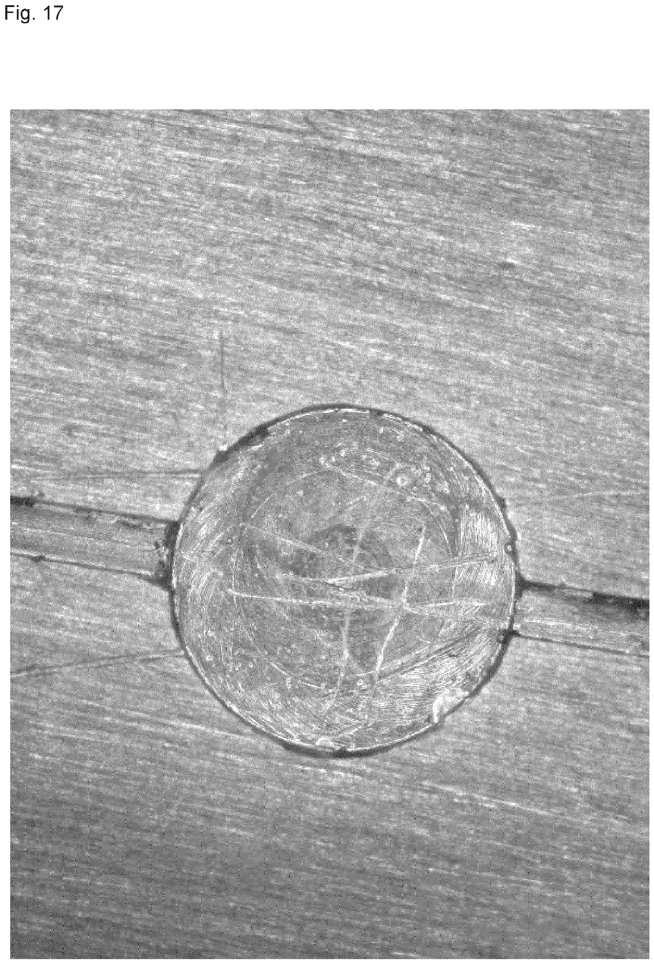

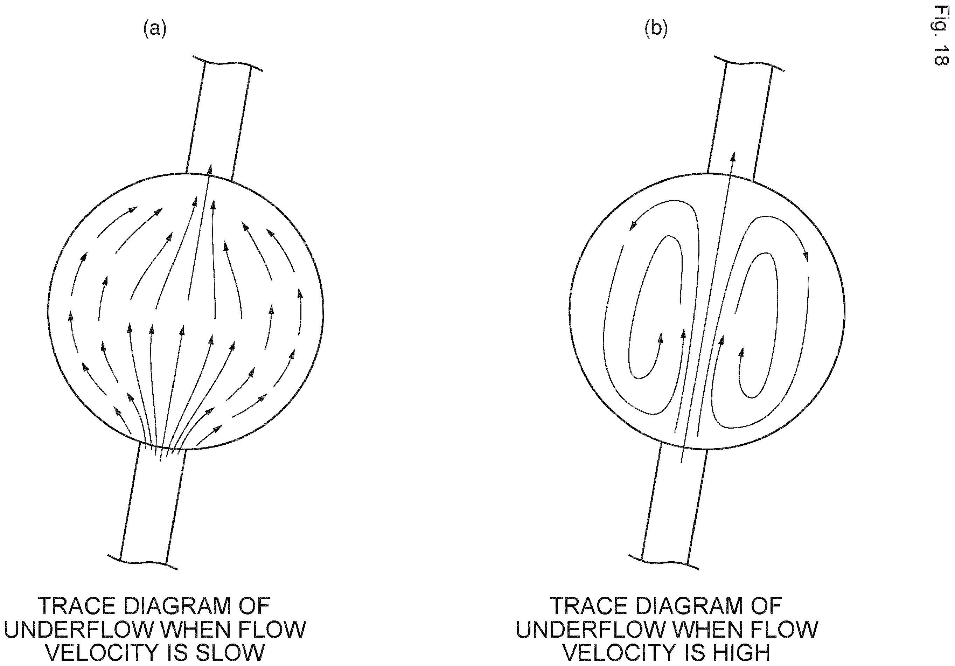

As shown in Fig. 17, a cell mass dissociator including a flow path having a narrow part and a wide part was produced. A liquid containing beads was poured into the cell dissociator shown in Fig. 17, and the movement of the beads was imaged. Fig. 18 shows a drawing in which the imaged movement of the beads was traced. It was shown that an underflow occurred in the wide part of the flow path from the movement of the beads.

### (Fourth example of second embodiment)

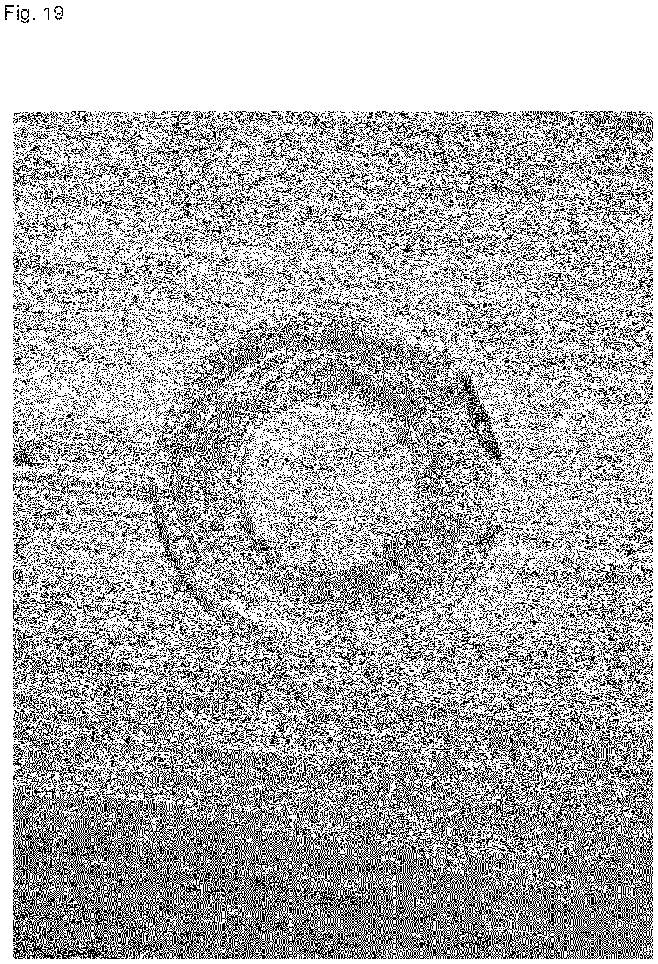

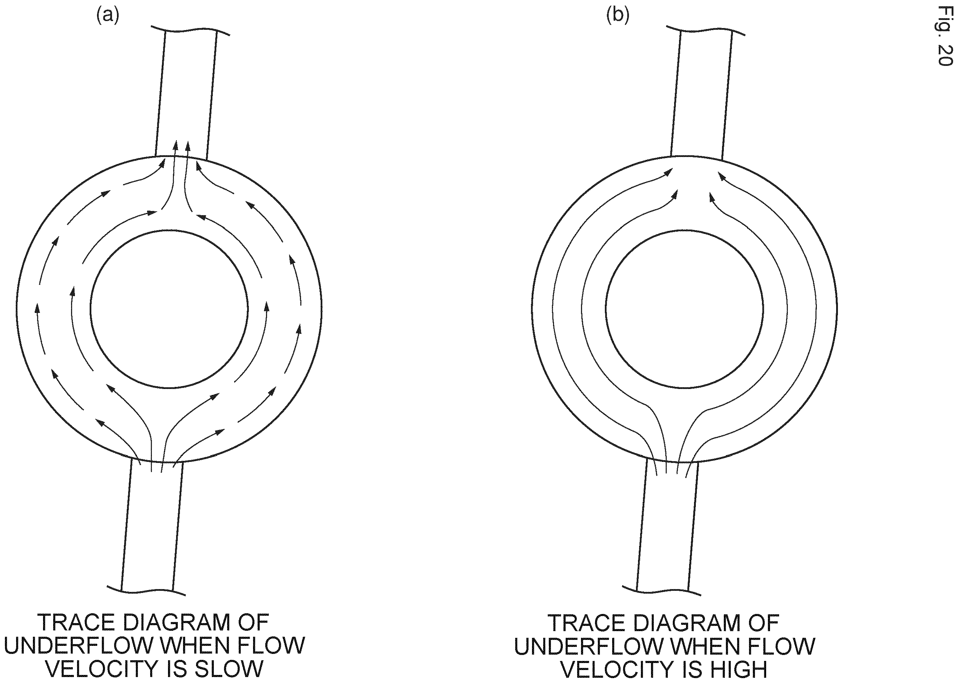

As shown in Fig. 19, a cell mass dissociator including a flow path which had a narrow part and a wide part and in which a member that partially prevented flow of a fluid in the flow path was provided in the wide part was produced. A liquid containing beads was poured into the cell dissociator shown in Fig. 19, and the movement of the beads was imaged. Fig. 20 shows a drawing in which the imaged movement of the beads was traced. It was shown that an underflow occurred in the wide part of the flow path from the movement of the beads.

### (Fifth example of second embodiment)

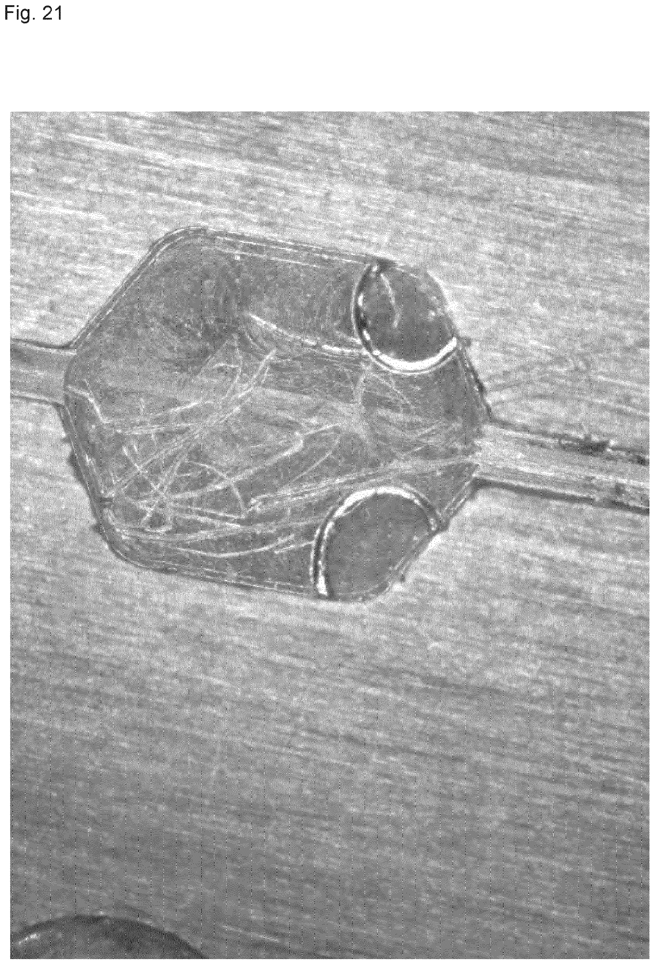

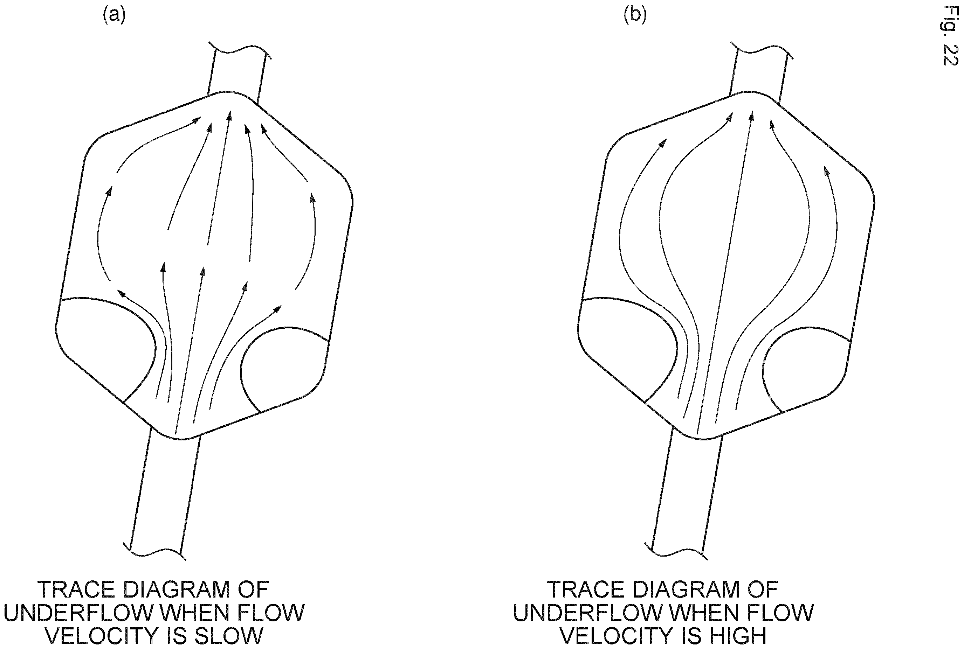

As shown in Fig. 21, a cell mass dissociator including a flow path which had a narrow part and a wide part and in which a member that partially prevented flow of a fluid in the flow path was provided in the wide part was produced. A liquid containing beads was poured into the cell dissociator shown in Fig. 21, and the movement of the beads was imaged. Fig. 22 shows a drawing in which the imaged movement of the beads was traced. It was shown that an underflow occurred in the wide part of the flow path from the movement of the beads.

### (Sixth example of second embodiment)

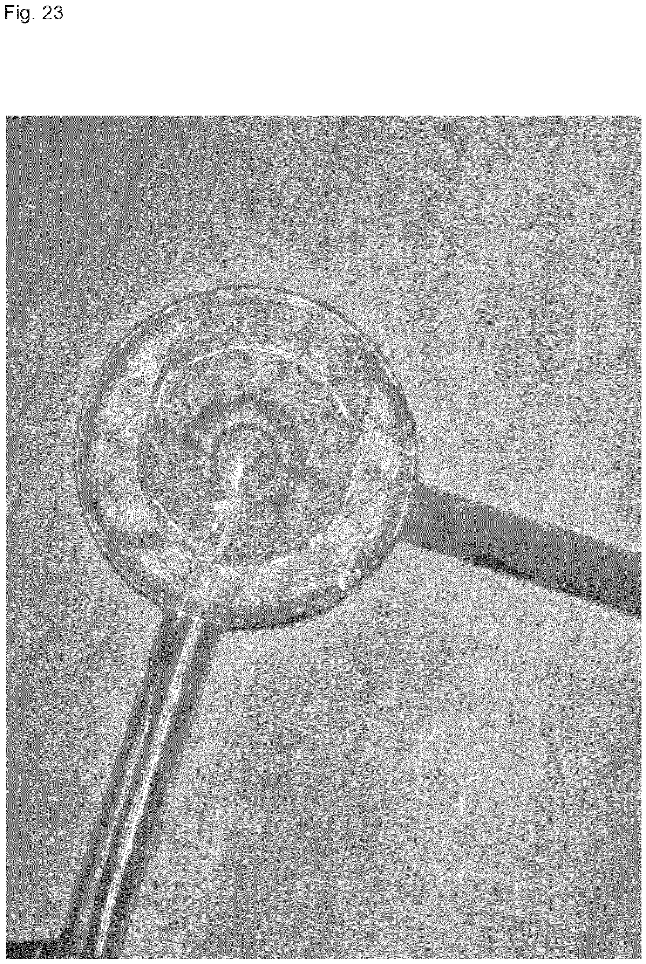

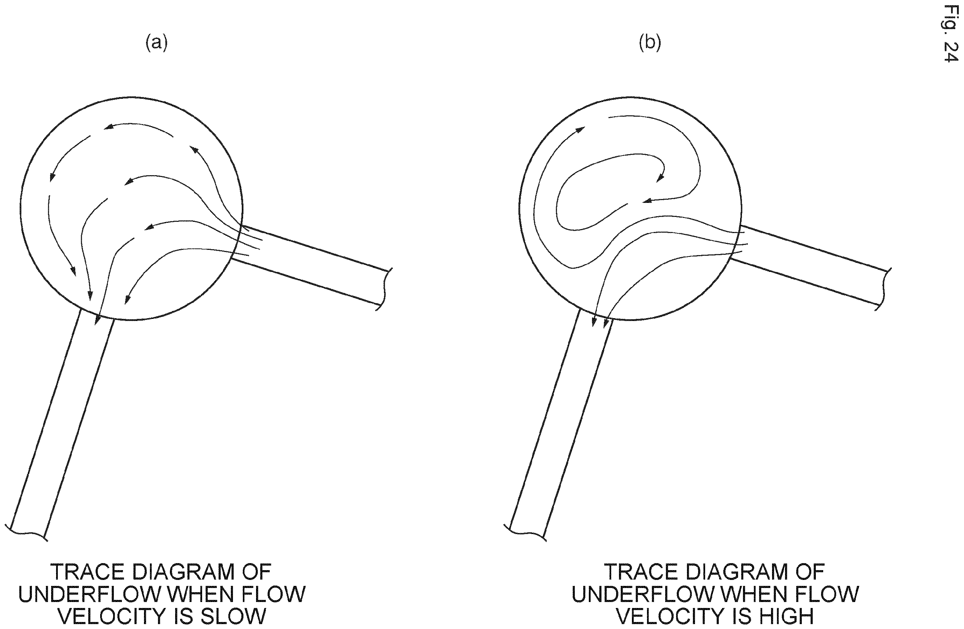

As shown in Fig. 23, a cell mass dissociator including a flow path having a narrow part and a wide part was produced. A liquid containing beads was poured into the cell dissociator shown in Fig. 23, and the movement of the beads was imaged. Fig. 24 shows a drawing in which the imaged movement of the beads was traced. It was shown that an underflow occurred in the wide part of the flow path from the movement of the beads.

### (Seventh example of second embodiment)

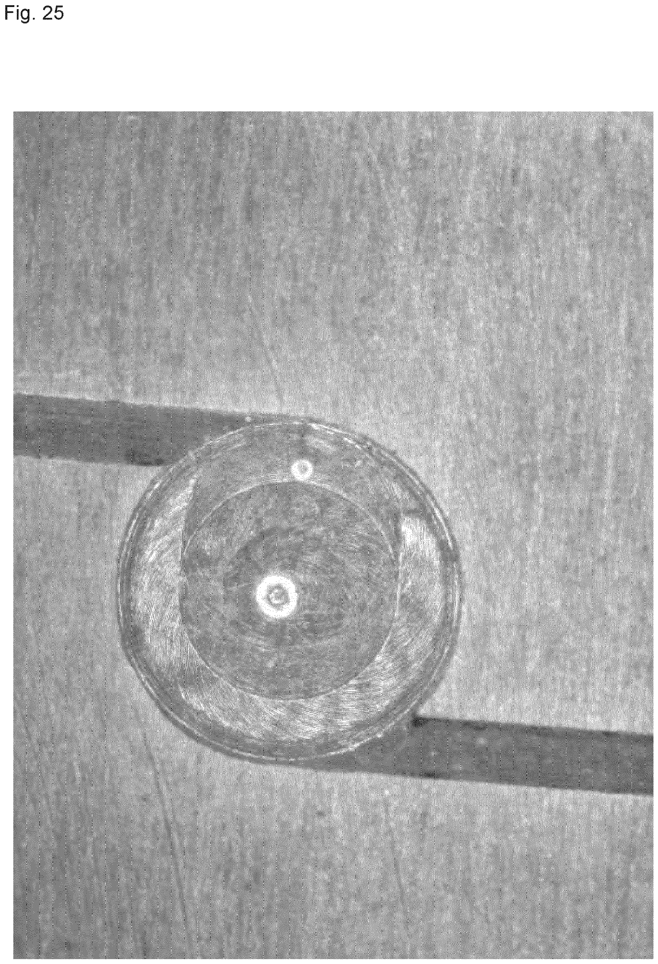

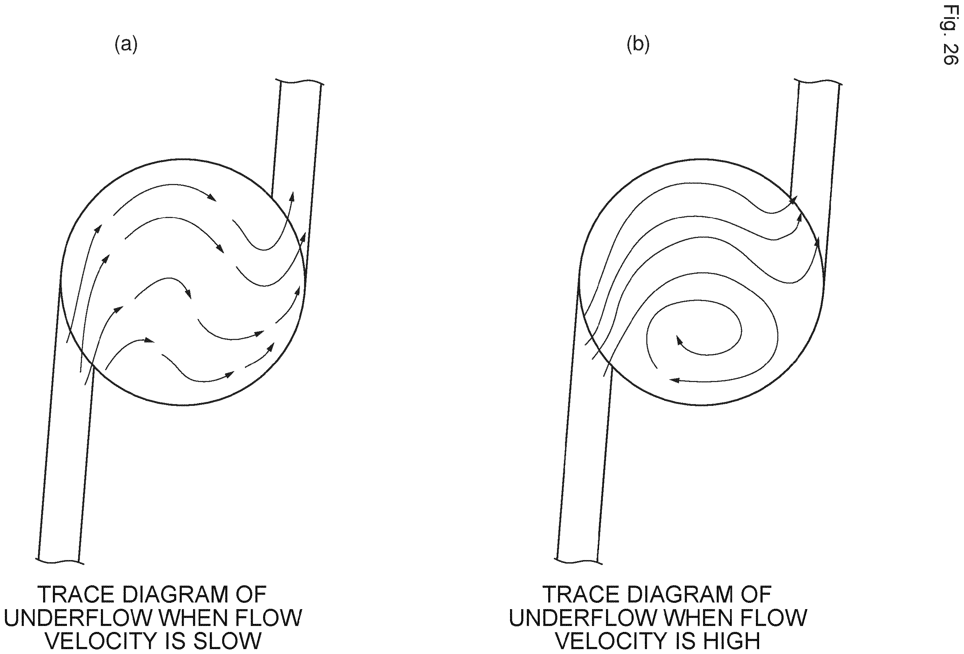

As shown in Fig. 25, a cell mass dissociator including a flow path having a narrow part and a wide part was produced. A liquid containing beads was poured into the cell dissociator shown in Fig. 25, and the movement of the beads was imaged. Fig. 26 shows a drawing in which the imaged movement of the beads was traced. It was shown that an underflow occurred in the wide part of the flow path from the movement of the beads.

### (Eighth example of second embodiment)

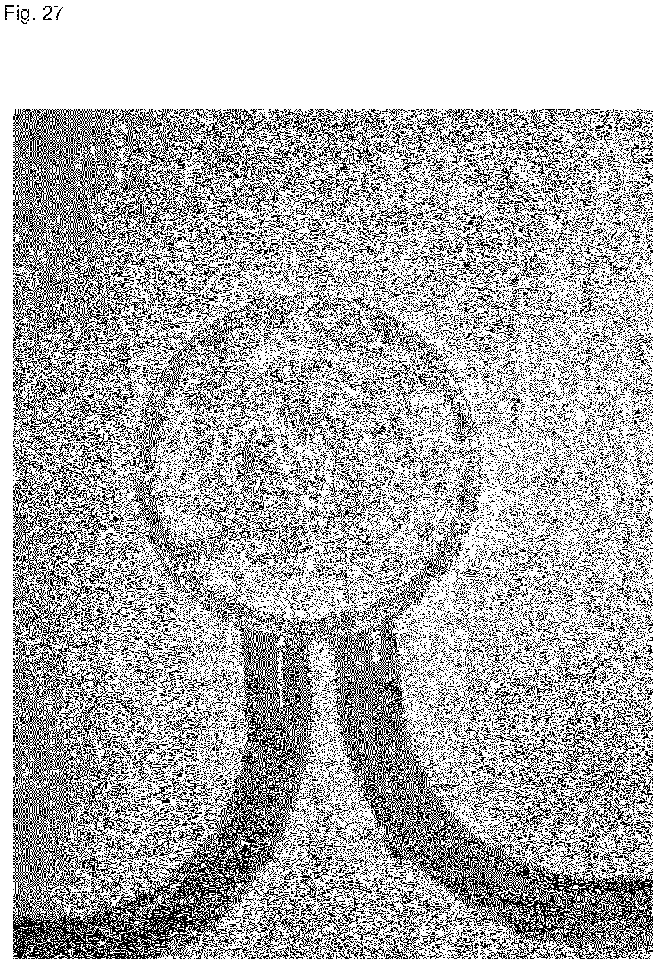

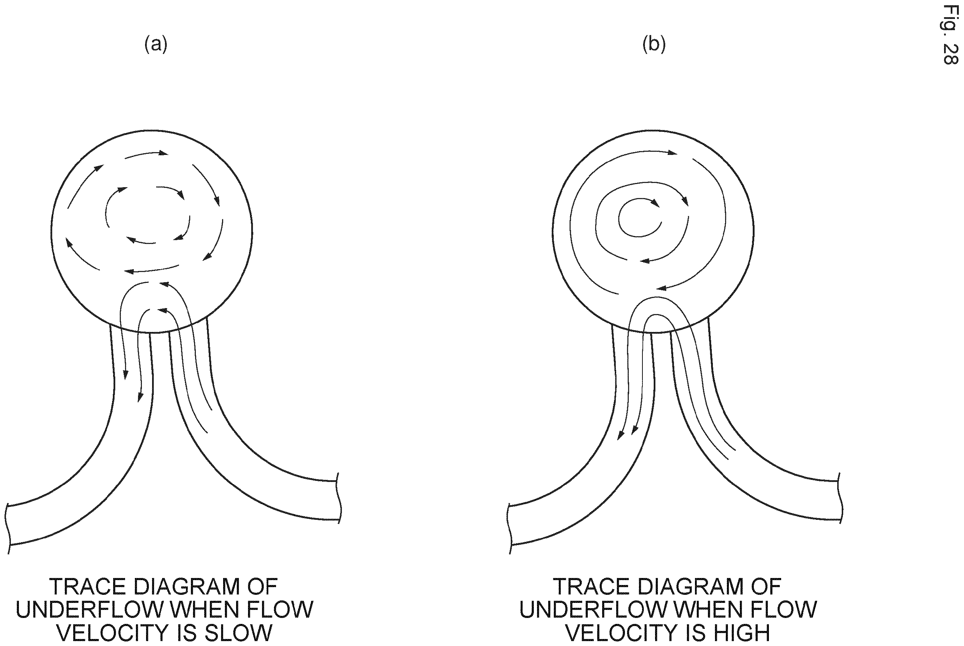

As shown in Fig. 27, a cell mass dissociator including a flow path having a narrow part and a wide part was produced. A liquid containing beads was poured into the cell dissociator shown in Fig. 27, and the movement of the beads was imaged. Fig. 28 shows a drawing in which the imaged movement of the beads was traced. It was shown that an underflow occurred in the wide part of the flow path from the movement of the beads.

### (Ninth example of second embodiment)

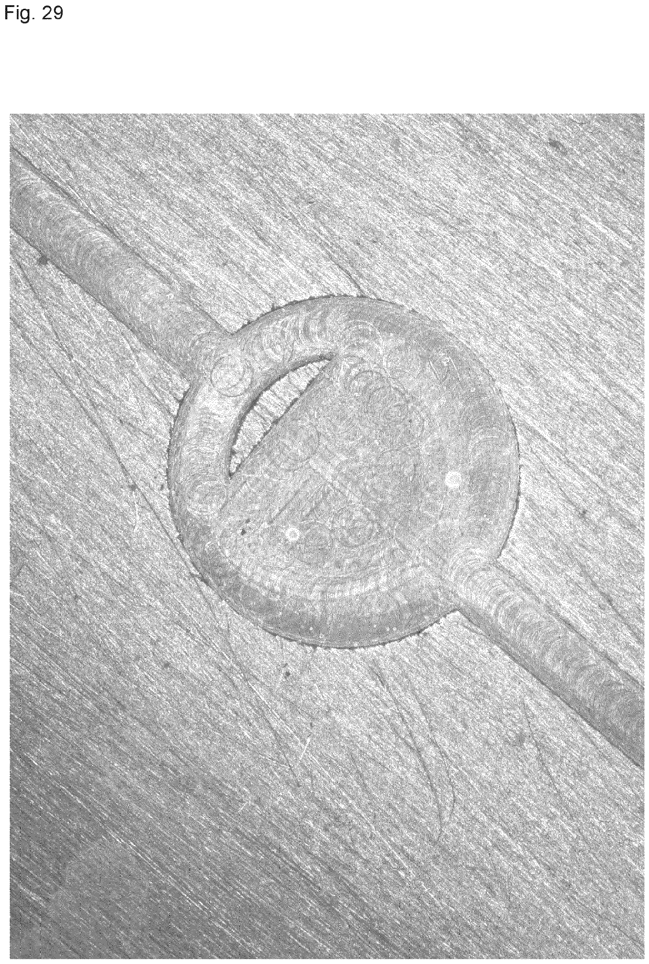

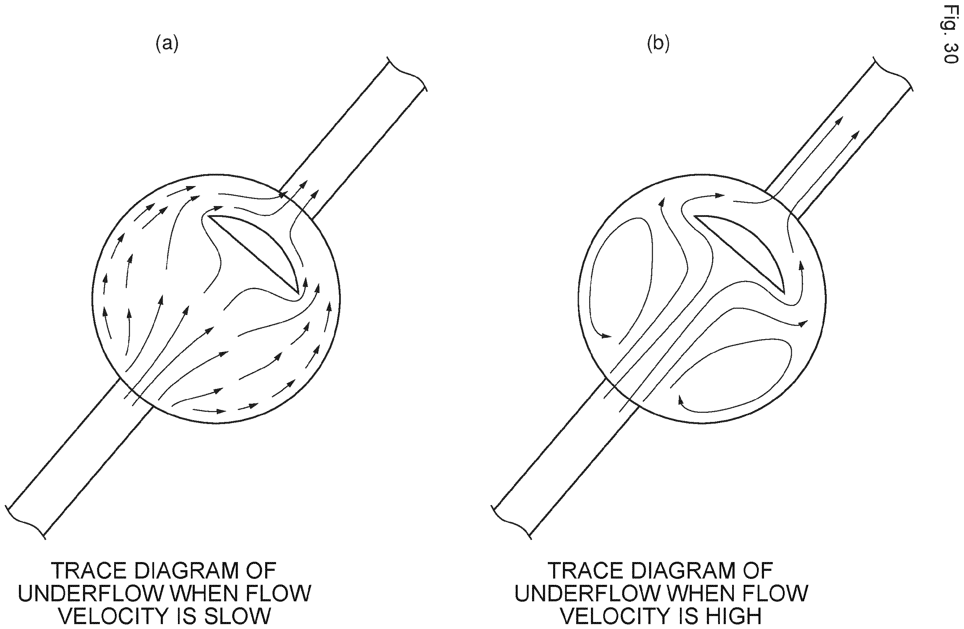

As shown in Fig. 29, a cell mass dissociator including a flow path which had a narrow part and a wide part and in which a member that partially prevented flow of a fluid in the flow path was provided in the wide part was produced. A liquid containing beads was poured into the cell dissociator shown in Fig. 29, and the movement of the beads was imaged. Fig. 30 shows a drawing in which the imaged movement of the beads was traced. It was shonw that an underflow occurred in the wide part of the flow path from the movement of the beads.

### (Other embodiments)

While the present invention has been described above with reference to the embodiments, it should be understood that the descriptions and drawings that form a part of this disclosure do not limit the present invention. It is apparent to those skilled in the art that various alternative embodiments, embodiments and operation techniques can be understood from this disclosure. For example, a filter assisting dissociation of the cell mass may be disposed in the flow path of the cell mass dissociator. Alternatively, a filter that passes a dissociated cell mass smaller than a predetermined size may be disposed in the flow path of the cell mass dissociator. The filter can be disposed in any part of the flow path. The filter may be disposed in the serpentine part of the flow path, disposed in a straight part of the flow path, disposed in the narrow part of the flow path, disposed in the wide part of the flow path, or disposed at the boundary between the narrow part and the wide part of the flow path. Accordingly, it should be understood that the present invention includes various embodiments and the like not described herein.

### Reference Signs List

- 10, 20: Flow path

## Claims

1. A cell mass dissociator comprising a serpentine flow path which is a flow path through which a cell mass flows.

2. The cell mass dissociator according to claim 1, wherein an underflow occurs in a fluid that flows through the flow path.

3. The cell mass dissociator according to claim 1 or 2, wherein the flow path is composed of a groove that is provided in a substrate and a lid that covers the groove.

4. The cell mass dissociator according to claim 1 or 2, wherein the flow path is embedded with an embedding member.

5. The cell mass dissociator according to claim 1 or 2, wherein the flow path is a hole that is provided in a solid member.

6. The cell mass dissociator according to any one of claims 1 to 5, wherein the flow path is able to be closed from the outside air.

7. The cell mass dissociator according to any one of claims 1 to 6, further comprising a fluid machine for causing the cell mass to flow through the flow path.

8. A cell mass dissociator comprising a flow path through which a cell mass flows, wherein
the flow path has a narrow part and a wide part that is wider than the narrow part, and
the flow path is able to be closed from the outside air.

9. The cell mass dissociator according to claim 8, wherein an underflow occurs in a fluid that flows through the flow path.

10. The cell mass dissociator according to claim 8 or 9, further comprising a member that is disposed in a wide part of the flow path and partially prevents a flow of a fluid in the flow path.

11. The cell mass dissociator according to any one of claims 8 to 10, wherein the flow path is composed of a groove that is provided in a substrate and a lid that covers the groove.

12. The cell mass dissociator according to any one of claims 8 to 10, wherein the flow path is embedded with an embedding member.

13. The cell mass dissociator according to any one of claims 8 to 10, wherein the flow path is a hole that is provided in a solid member.

14. The cell mass dissociator according to any one of claims 8 to 13, further comprising a fluid machine for causing the cell mass to flow through the flow path.

15. A method for manufacturing a cell mass dissociator, comprising forming a serpentine flow path through which a cell mass flows.

16. The method for manufacturing a cell mass dissociator according to claim 15, wherein a groove is provided in a substrate to form the flow path.

17. The method for manufacturing a cell mass dissociator according to claim 15 or 16, further comprising embedding the flow path with an embedding member.

18. The method for manufacturing a cell mass dissociator according to claim 15, wherein a hole is provided in a solid member to form the flow path.

19. The method for manufacturing a cell mass dissociator according to any one of claims 15 to 18, wherein the flow path is formed by an optical shaping method.

20. A method for manufacturing a cell mass dissociator, comprising:
forming a flow path having a narrow part and a wide part that is wider than the narrow part, which is a flow path through which a cell mass flows; and
closing the flow path from the outside air.

21. The method for manufacturing a cell mass dissociator according to claim 20, further comprising providing a member that partially prevents a flow of a fluid in the flow path in the wide part of the flow path.

22. The method for manufacturing a cell mass dissociator according to claim 20 or 21, wherein the flow path is composed of a groove that is provided in a substrate and a lid that covers the groove.

23. The method for manufacturing a cell mass dissociator according to any one of claims 20 to 22, wherein a groove is provided in a substrate to form the flow path.

24. The method for manufacturing a cell mass dissociator according to any one of claims 20 to 22, further comprising embedding the flow path with an embedding member.

25. The method for manufacturing a cell mass dissociator according to any one of claims 20 to 22, wherein a hole is provided in a solid member to form the flow path.

26. The method for manufacturing a cell mass dissociator according to any one of claims 20 to 25, wherein the flow path is formed by an optical shaping method.

27. A method for dissociating a cell mass, comprising causing a cell mass to flow through a serpentine flow path and dissociating the cell mass.

28. The method for dissociating a cell mass according to claim 27, wherein an underflow occurs in a fluid containing the cell mass in the flow path.

29. The method according to claim 27 or 28, wherein the cell mass that flows through the flow path is contained in a cryopreservation solution.

30. The method according to any one of claims 27 to 29, further comprising bringing the cell mass into contact with a cell detachment solution before the cell mass flows through the serpentine flow path.

31. A method for dissociating a cell mass, comprising causing a cell mass to flow through a flow path of a cell mass dissociator which has the flow path through which a cell mass flows and in which the flow path has a narrow part and a wide part that is wider than the narrow part, and the flow path is able to be closed from the outside air, and dissociating the cell mass.

32. The method for dissociating a cell mass according to claim 31, wherein an underflow occurs in a fluid containing the cell mass in the flow path.

33. The method for dissociating a cell mass according to claim 31 or 32, wherein a member that partially prevents a flow of a fluid in the flow path is provided in the wide part of the flow path.

34. The method according to any one of claims 31 to 33, wherein the cell mass that flows through the flow path is contained in a cryopreservation solution.

35. The method according to any one of claims 31 to 34, further comprising bringing the cell mass into contact with a cell detachment solution before the cell mass flows through the flow path.
